Europäisches Patentamt

European Patent Office

Office européen des brevets

(19)

(11) **EP 1 346 743 A1**

(12) **EUROPÄISCHE PATENTANMELDUNG**

(43) Veröffentlichungstag:
**24.09.2003 Patentblatt 2003/39**

(51) Int Cl.⁷: **A61M 16/00**

(21) Anmeldenummer: **03002456.6**

(22) Anmeldetag: **05.02.2003**

(84) Benannte Vertragsstaaten:
**AT BE BG CH CY CZ DE DK EE ES FI FR GB GR
HU IE IT LI LU MC NL PT SE SI SK TR**
Benannte Erstreckungsstaaten:
**AL LT LV MK RO**

(30) Priorität: **21.03.2002 DE 10212497**

(71) Anmelder: **Gottlieb Weinmann Geräte für Medizin
und Arbeitsschutz GmbH + Co.
22525 Hamburg (DE)**

(72) Erfinder: **Tiemann, Björn
20535 Hamburg (DE)**

(74) Vertreter: **Klickow, Hans-Henning
Patentanwälte
Hansmann-Klickow-Hansmann
Jessenstrasse 4
22767 Hamburg (DE)**

(54) **Verfahren zur Steuerung eines Beatmungsgerätes sowie Vorrichtung zur Beatmung**

(57)    Das Verfahren dient zur Steuerung eines Beatmungsgerätes, das eine Abgabesteuerung (19) für Atemgas aufweist. Die Abgabesteuerung (19) ist an einen Sensor (20) für ein Meßsignal angeschlossen und ein Druckaufbau wird in Abhängigkeit von einem zum Meßsignal korrespondierenden Triggersignal durchgeführt. Das Triggersignal wird mit einem Bezugswert verglichen und der Druck wird verändert, wenn das Triggersignal mindestens gleich dem Bezugswert ist. Der Bezugswert wird in Abhängigkeit von einem zeitlichen Verlauf verändert. Die Abgabesteuerung (19) ist mit einem Stellelement zur Beeinflussung einer Atemgasströmung verbunden. Die Veränderung des Bezugswertes wird durch einen Bezugswertadapter (24) durchgeführt.

FIG.4

EP 1 346 743 A1

## Beschreibung

Die Erfindung betrifft ein Verfahren zur Steuerung eines Beatmungsgerätes, bei dem ein Druckaufbau in Abhängigkeit von einem Triggersignal durchgeführt wird, das mit einem Bezugswert verglichen wird und bei dem der Druck verändert wird, wenn das Triggersignal mindestens gleich dem Bezugswert ist.

Die Erfindung betrifft darüber hinaus eine Vorrichtung zur Beatmung, die eine Abgabesteuerung für Atemgas aufweist und bei der die Abgabesteuerung an einen Sensor für ein Meßsignal angeschlossen und mit einer Triggersignalauswertung versehen ist, die ein zum Meßsignal korrespondierendes Triggersignal mit einem Bezugswert vergleicht, sowie bei der die Abgabesteuerung mit einem Stellelement zur Beeinflussung einer Atemgasströmung verbunden ist.

Bei einem Betrieb von Beatmungsgeräten wird häufig angestrebt, eine Optimierung der Betriebsweise zu erreichen. Beispielsweise besteht bei Geräten zur Durchführung einer Sauerstofftherapie insbesondere bei einem mobilen Betrieb die Anforderung, eine während jedes Atemzyklus durchgeführte Sauerstoffabgabe zeitlich zum Verlauf der Atmungsperiode derart zu optimieren, daß mit einer möglichst geringen Menge an abgegebenem Sauerstoff der gewünschte Therapieeffekt erreicht werden kann.

Bei einer Optimierung der Ansteuerung des Beatmungsgerätes kann hierdurch als Folge der Optimierung der Sauerstoffabgabe eine Verlängerung der Gerätebetriebsfähigkeit insbesondere bei mobilen Anwendungen erreicht werden.

Ein weiteres Anwendungsgebiet besteht darin, bei einem Betrieb von stationären Beatmungsgeräten, beispielsweise für eine Versorgung von COPD-Patienten, eine möglichst genaue Adaption der künstlich erzeugten Atemgasströmung an einen natürlichen Atemrhythmus des Patienten vorzunehmen. Hierdurch kann vermieden werden, daß das Beatmungsgerät gegen den natürlichen Atmungsrhythmus des Patienten arbeitet. Eine derartige Arbeitsweise ist zum einen gerätetechnisch unerwünscht, darüber hinaus empfindet ein Patient ein asynchrones Arbeiten des Beatmungsgerätes auch als unangenehm.

Zu einer Synchronisation des Betriebes des Beatmungsgerätes an einen natürlichen Atmungsrhythmus des Patienten ist es bereits bekannt, meßtechnisch einen Muskelimpuls des Zwerchfells des Patienten zu erfassen und als Triggersignal für das Beatmungsgerät zu verwenden, um einen Druckaufbau auszulösen. Bei einer derartigen meßtechnischen Erfassung hat in der Regel der Patient aber bereits bis zu 30% seiner eigenen Atmungsarbeit geleistet, bis eine synchrone Gerätesteuerung erfolgen kann.

Die bekannten Verfahren zur Ansteuerung von Beatmungsgeräten synchron zu einer spontanen Atmungsaktivität eines Patienten erfordern eine relativ hohe Triggerschwelle. Eine Absenkung der Triggerschwelle führt in der Regel aufgrund des Auftretens von Störsignalen zu erheblichen Schwierigkeiten, da Fehltriggerungen als Folge einer zu niedrigen Triggerschwelle Fehlsteuerungen verursachen können.

Aufgabe der vorliegenden Erfindung ist es daher, ein Verfahren der einleitend genannten Art derart zu verbessern, daß eine Absenkung einer Triggerschwelle bei gleichzeitiger Vermeidung einer erhöhten Störungsanfälligkeit unterstützt wird.

Diese Aufgabe wird erfindungsgemäß dadurch gelöst, daß der Bezugswert in Abhängigkeit von einem zeitlichen Verlauf verändert wird.

Weitere Aufgabe der vorliegenden Erfindung ist es, eine Vorrichtung der einleitend genannten Art derart zu konstruieren, daß ein für den Patienten angenehmeres Betriebsverhalten bereitgestellt wird.

Diese Aufgabe wird erfindungsgemäß dadurch gelöst, daß die Triggersignalauswertung eine Bezugswertadaption zur zeitlichen Veränderung des Bezugswertes in Abhängigkeit von einem zeitlichen Verlauf aufweist.

Durch die zeitliche Veränderung des Bezugswertes ist es möglich, eine Adaption an einen typischen Atmungsrhythmus durchzuführen. Insbesondere kann hierbei ausgenutzt werden, daß beispielsweise zu einem Beginn einer Einatmungsphase eine Fortsetzung der Einatmungsphase wesentlich wahrscheinlicher als ein vorzeitiges Ende der Einatmungsphase ist und daß kurz vor einem Ende der Ausatmungsphase mit hoher Wahrscheinlichkeit ein erneuter Beginn der Einatmungsphase eine hohe Wahrscheinlichkeit aufweist.

In Abhängigkeit von der Wahrscheinlichkeit des Auftretens des die jeweilige Funktion des Beatmungsgerätes auslösenden Ereignisses kann somit der Bezugswert mit der Triggerschwelle verändert werden. Grundsätzlich gilt hierbei, daß mit einer zunehmenden Wahrscheinlichkeit für das Auftreten des auslösenden Ereignisses die Triggerschwelle abgesenkt werden kann, da der Einfluß von Störungen zum einen unwahrscheinlicher wird und darüber hinaus selbst bei einer Fehlauslösung aufgrund einer auftretenden Störung das Resultat einer Fehltriggerung aufgrund der zeitlichen Nähe zu einem korrekten Triggerzeitpunkt deutlich weniger störend ist als bei einer Fehlauslösung zu einem völlig falschen Zeitpunkt.

Eine Adaption an einen sich ändernden Atmungsrhythmus kann dadurch erfolgen, daß die zeitliche Veränderung des Bezugswertes während der Dauer von zeitlich relativ aufeinander folgenden Beatmungszyklen durchgeführt wird.

Zur Anpassung an die Dynamik aufeinander folgender Inspirations- und Exspirationsphasen wird vorgeschlagen, daß die Veränderung des Bezugswertes während der Dauer eines Beatmungszyklus durchgeführt wird. ,

Eine Ausnutzung sich ändernder Ereigniswahrscheinlichkeiten kann dadurch erfolgen, daß ein Bezugswert zur Detektion einer Exspirationsphase vor einem erwarteten Beginn der Exspirationsphase abgesenkt wird.

Ebenfalls ist daran gedacht, daß ein Bezugswert zur Detektion einer Inspirationsphase vor einem erwarteten Beginn der Inspirationsphase abgesenkt wird.

Eine mathematisch einfache funktionelle Abhängigkeit wird dadurch bereitgestellt, daß der Bezugswert in Abhängigkeit von einem linearen Wahrscheinlichkeitsverlauf verändert wird.

Darüber hinaus ist auch daran gedacht, daß der Bezugswert in Abhängigkeit von einem nicht linearen Wahrscheinlichkeitsverlauf verändert wird.

Eine Adaption an einen sich ändernden Atmungsrhythmus bei gleichzeitig ruhigem Systemverhalten kann dadurch erfolgen, daß der Bezugswert in Abhängigkeit von einer Mittelwertbildung verändert wird.

Zur Berücksichtigung von sich wiederholenden periodischen Abläufen wird vorgeschlagen, daß zur Veränderung des Bezugswertes eine erfaßte Dauer von mindestens zwei Inspirationsphasen ausgewertet wird.

Ebenfalls ist daran gedacht, daß zur Veränderung des Bezugswertes eine erfaßte Dauer von mindestens zwei Exspirationsphasen ausgewertet wird.

Eine vergrößerte Adaptionsgenauigkeit kann dadurch bereitgestellt werden, daß zur Veränderung des Bezugswertes eine Streuung mindestens eines Meßwertes ausgewertet wird.

Zur Einhaltung eines vorgegebenen Betriebsbereiches wird vorgeschlagen, daß eine adaptive Veränderung des Bezugswertes ausschließlich innerhalb vorgegebener Variationsgrenzen durchgeführt wird.

Zur Vermeidung einer zu großen Systemempfindlichkeit wird vorgeschlagen, daß der Bezugswert höchstens bis zu einem minimalen Triggerwert abgesenkt wird.

Eine Deaktivierung des Systems kann dadurch vermieden werden, daß der Bezugswert höchstens bis zu einem maximalen Triggerwert erhöht wird.

Eine Adaption bei der Auswertung komplexer Verlaufskriterien kann dadurch erfolgen, daß zur Änderung des Bezugswertes ein neuronales Netz verwendet wird.

Ebenfalls ist daran gedacht, daß zur Änderung des Bezugswertes eine Fuzzy-Logik verwendet wird.

Eine typische Anwendung besteht darin, daß die adaptive Veränderung des Bezugswertes im Zusammenhang mit dem Betrieb eines Beatmungsgerätes für CPAP-Anwendungen durchgeführt wird.

Ein weiteres Anwendungsgebiet wird dadurch erschlossen, daß die adaptive Veränderung des Bezugswertes im Zusammenhang mit dem Betrieb eines Beatmungsgerätes für COPD-Anwendungen durchgeführt wird.

Ein weiteres Anwendungsgebiet wird dadurch bereitgestellt, daß die adaptive Veränderung des Bezugswertes im Zusammenhang mit dem Betrieb eines Beatmungsgerätes für Sauerstofftherapien durchgeführt wird.

Eine einfache meßtechnische Erfassung wird dadurch unterstützt, daß bei der adaptiven Veränderung des Bezugswertes Druckmeßwerte ausgewertet werden.

Eine weitere Meßvariante besteht darin, daß bei der adaptiven Veränderung des Bezugswertes Strömungsmengenmeßwerte ausgewertet werden.

In den Zeichnungen sind Ausführungsbeispiele der Erfindung schematisch dargestellt. Es zeigen:

Fig. 1    Eine perspektivische Prinzipdarstellung eines Beatmungsgerätes mit Verbindungsschlauch zu einer Beatmungsmaske,

Fig. 2    einen zeitlichen Verlauf des Volumenflusses des Atemgases während aufeinander folgender Einatmungs- sowie Ausamtungsphasen sowie einen zugeordneten Wahrscheinlichkeitsverlauf,

Fig. 3    einen zeitlichen Verlauf eines auslösenden Triggersignals sowie eines zeitlich veränderlichen Bezugswertes als inspiratorische und exspiratorische Triggerschwelle,

Fig. 4    ein schematisches Blockschaltbild zur Veranschaulichung der funktionellen Komponenten der Gerätesteuerung und

Fig. 5    ein schematisches Ablaufdiagramm zur Veranschaulichung des Ablaufes der Gerätesteuerung.

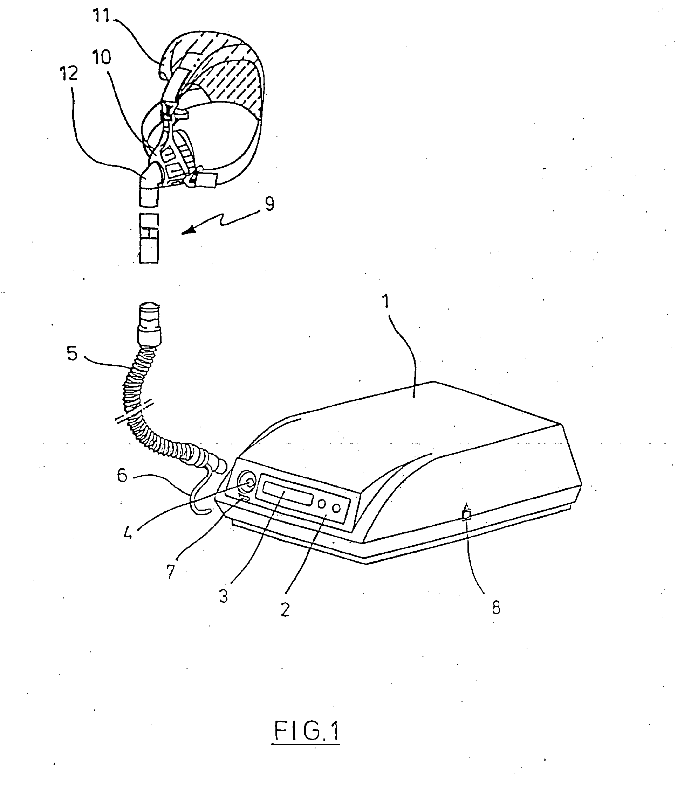

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement angeordnet.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

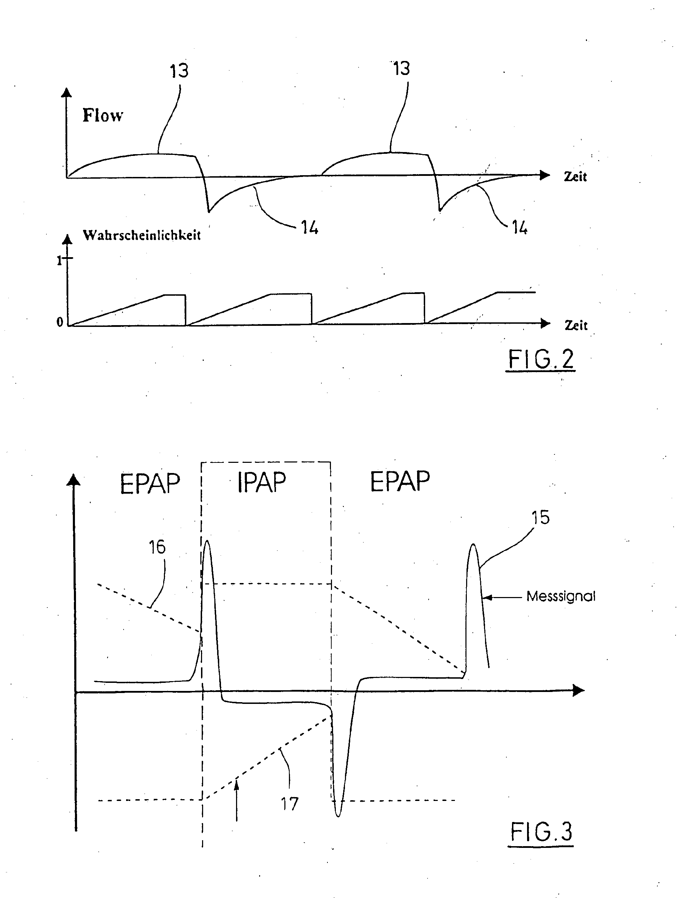

Fig. 2 veranschaulicht den Verlauf des Volumenflusses der Atemgasströmung. Es folgen jeweils Exspirationsphasen (13) und Inspirationsphasen (14) zyklisch aufeinander. Fig. 2 zeigt im unteren Teil ein weiteres Diagramm, in dem die zugehörige Wahrscheinlichkeit über der Zeit aufgetragen ist. Bei dem dargestellten Beispiel erfolgt der zeitliche Anstieg der Wahrscheinlichkeit linear, da hier als Adaptionskriterium eine linear zunehmende Wahrscheinlichkeit für einen erneuten Atemphasenwechsel während des zeitlichen Verlaufs einer aktuellen Atemphase zugrunde gelegt wird. Es ist aber auch möglich, einen quadratischen oder exponentiellen Verlauf zu verwenden.

Die Steigung der linearen Rampen des Wahrscheinlichkeitsverlaufes ergibt sich gemäß einem Ausführungsbeispiel durch eine Mittelwertbildung aus den zuvor ermittelten Atemzeiten der Exspirationsphasen (13) sowie der Inspirationsphasen (14). Eine maximale Empfindlichkeit des Triggers wird bei einer vollständig regelmäßigen Atmung exakt zum voraussichtlichen Triggerzeitpunkt erreicht. Durch die Mittelwertbildung erfolgt eine Adaption an unterschiedliche Atmungszustände. Ab einer vorgebbaren maximalen Wahrscheinlichkeit erfolgt keine Erhöhung des Wahrscheinlichkeitswertes. Die maximale Wahrscheinlichkeit beträgt im dargestellten Ausführungsbeispiel etwa 0,4 bis 0,5.

Fig. 3 veranschaulicht den zeitlichen Verlauf eines den Trigger auslösenden Signals (15)-eines inspiratorischen Bezugwertes (16) als Triggerschwelle sowie eines exspiratorischen Bezugswertes (17) als Triggerschwelle.

Als den Trigger auslösendes Signal (15) können entweder unmittelbar meßtechnisch erfaßte Signalverläufe oder aus derartigen Signalverläufen abgeleitete zeitliche Verläufe verwendet werden. Beim Ausführungsbeispiel gemäß Fig. 3 wird das dargestellte Signal (15) als dritte Potenz der ersten Ableitung eines gemessenen Signals zur jeweiligen Durchflußmenge an Atemgas ermittelt.

Eine weitere Adaption des Wahrscheinlichkeitsverlaufes kann aus einer Ermittlung der kurzfristigen Streuung der Inspirationszeiten sowie der Exspirationszeiten gewonnen werden. Bei einer großen Streuung liegt eine unregelmäßige Atmung vor, so daß sowohl eine Störungswahrscheinlichkeit erhöht als auch die Auswirkungen von fehlerhaften Triggerauslösungen vergrößert werden. Bei einer Ermittlung derartiger zugehöriger Meßwertverläufe erfolgt deshalb eine Erhöhung der Triggerschwelle und damit eine Verringerung der Triggerempfindlichkeit.

Darüber hinaus ist es möglich, gerätetechnisch einen jeweiligen Adaptionsbereich vorzugeben. Es können hierdurch sowohl obere und untere Grenzen für die adaptive Veränderung der Triggerschwelle als auch eine zeitliche Vorgabe für eine Änderungsgeschwindigkeit erfolgen. Ein jeweiliger Bezugswert als Triggerschwelle T, mit dem das Triggersignal verglichen wird, errechnet sich nach der Formel

$$T(t) = -FA * FS * (MaxT - MinT) * \frac{t}{t_{exspected}} + MaxT$$

In der obigen Formel bedeuten:

T: Triggerschwelle
FA: Wirkungsgrad (Bestimmt durch den Arzt)
FS: Korrekturfaktor Streuung (proportional zur Streuung der letzten Insp/Exspirationszeiten)
MaxT, MinT: Maximale und minimale Triggerschwelle
Texspected: Erwarteter Zeitraum bis zum nächsten Atemphasenwechsel (getrennt nach Inspiration und Exspiration)

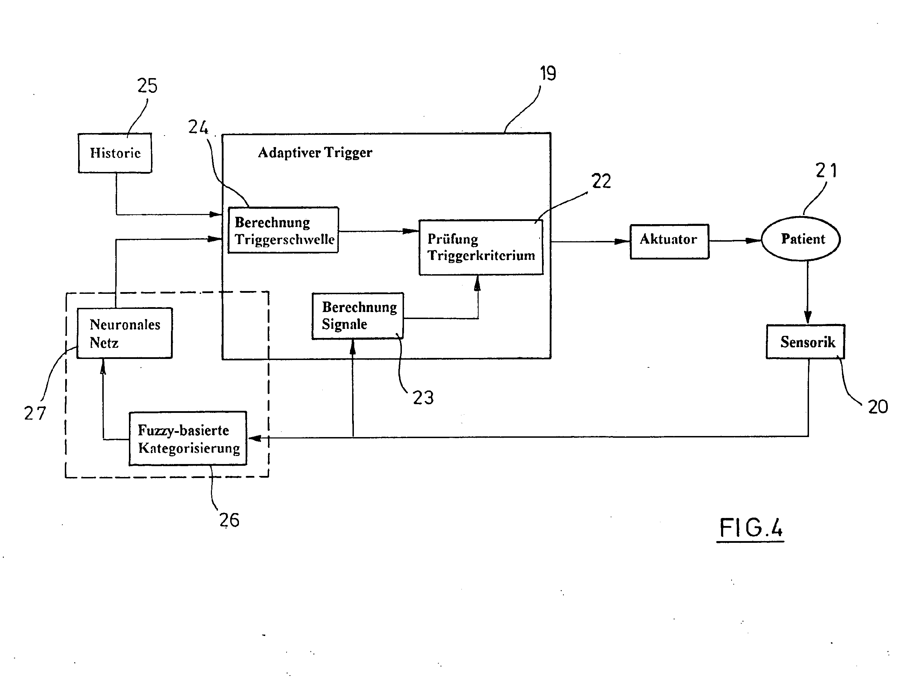

Fig. 4 veranschaulicht eine funktionelle Struktur zur Durchführung des Steuerungsverfahrens. Eine Einstellung der jeweiligen Atemgasströmung erfolgt unter Verwendung eines Aktuators (18), der an eine Abgabesteuerung (19) angeschlossen ist. Über einen Sensor (20) wird ein Meßsignal im Bereich eines Patienten (21), ein Druck- oder Flußsignal im Bereich einer Beatmungsmaske oder ein entsprechendes Meßsignal im Bereich eines Beatmungsschlauches oder innerhalb des Beatmungsgerätes selbst erfaßt. Der Sensor (20) ist an die Abgabesteuerung (19) angeschlossen.

Die Abgabesteuerung (19) weist eine Triggersignalauswertung (22) auf, der ein Bezugswert sowie das Sensorsignal zugeführt wird. Alternativ zu einer unmittelbaren Berechnung des Signals des Sensors (20) ist es auch möglich, eine Signalaufbereitung (23) zwischenzuschalten. Der Bezugswert wird von einem Bezugswertadapter (24) an einen zeitlichen Verlauf der Exspirationsphasen (13) sowie der Inspirationsphasen (14) angepaßt. Der Bezugswertadapter (24) ist an einen Datenspeicher (25) angeschlossen, der Verlaufsdaten zu bereits erfolgten Beatmungszyklen bereitstellt. Eine weitere Adaption der Triggerung kann beispielsweise durch eine Fuzzy-Logik (26) und / oder durch ein neuronales Netz (27) erfolgen.

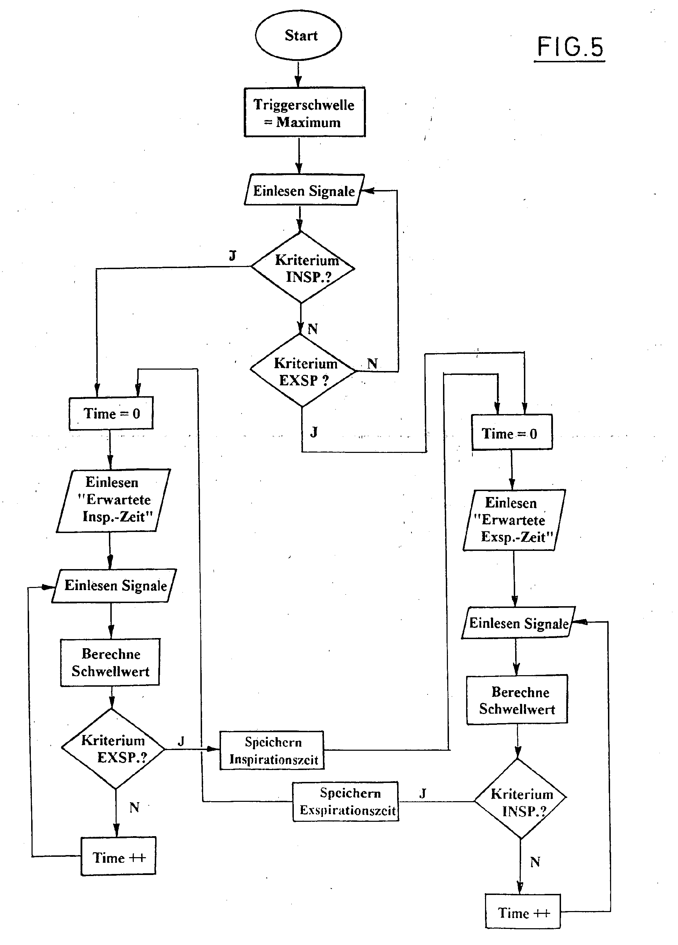

Fig. 5 zeigt beispielhaft einen zeitlichen Ablauf bei der Durchführung des Steuerungsverfahrens. Bei einem Einschalten des Systems wird zunächst der Bezugswert bzw. die Triggerschwelle auf einen vorgegebenen Maximalwert gesetzt. Anschließend erfolgt ein Einlesen von Meßwerten des Sensors (20). Liegt ein Erfassungskriterium für die Detektion einer Inspirationsphase (14) vor, so wird in eine entsprechende Inspirationsverarbeitung verzweigt. Liegt das Kriterium für die Erkennung einer Exspirationsphase (13) vor, so wird in die zugehörige Exspirationsverarbeitung verzweigt. Sind beide Kriterien nicht erfüllt, so werden erneut Signale des Sensors (20) eingelesen.

Bei der Durchführung der Steuerung zur Inspirationsphase (14) wird zunächst ein Timer auf null gesetzt und es wird ein Wert für eine erwartete Inspirationszeit eingelesen. Anschließend werden Meßwerte des Sensors (20) ausgewertet und hieraus wird ein aktueller Bezugswert als Triggerschwelle berechnet. Liegt ein Auslösekriterium zur Detektion einer Exspirationsphase (13) vor, so wird nach einer zuvor erfolgten Abspeicherung der aktuellen Inspirationszeit in die Verarbeitung für die Exspirationsphase (13) verzweigt. Liegt das Kriterium zur Erfassung einer Exspirationsphase (13) noch nicht vor, so wird der Wertestand des Timers für die Inspirationsphase erhöht und der Ablauf wird erneut durchlaufen.

Nach dem Erkennen des Kriteriums für den Beginn einer Exspirationsphase (13) sowie der Abspeicherung der aktuellen Inspirationszeit erfolgt während der Signalverarbeitung für die Exspirationsphase (13) sinngemäß der gleiche Ablauf, der eben bereits für die Inspirationsphase (14) beschrieben wurde. Nach einem Erkennen des Kriteriums für den Beginn einer neuen Inspirationsphase (14) der Ablauf der Signalverarbeitung für die Exspirationsphase (13) beendet und erneut die Verarbeitung für eine Inspirationsphase (14) durchlaufen. Wechselseitig erfolgt somit eine Ermittlung der aktuellen Inspirations- sowie Exspirationsdauern.

**Patentansprüche**

1. Verfahren zur Steuerung eines Beamtungsgerätes, bei dem ein Druckaufbau in Abhängigkeit von einem Triggersignal durchgeführt wird, das mit einem Bezugswert verglichen wird und bei dem der Druck verändert wird, wenn das Triggersignal mindestens gleich dem Bezugswert ist, **dadurch gekennzeichnet, daß** der Bezugswert in Abhängigkeit von einem zeitlichen Verlauf verändert wird.

2. Verfahren nach Anspruch 1, **dadurch gekennzeichnet, daß** die zeitliche Veränderung des Bezugswertes während der Dauer von zeitlich relativ aufeinander folgenden Beatmungszyklen durchgeführt wird.

3. Verfahren nach Anspruch 1, **dadurch gekennzeichnet, daß** die Veränderung des Bezugswertes während der Dauer eines Beatmungszyklus durchgeführt wird.

4. Verfahren nach einem der Ansprüche 1 bis 3, **dadurch gekennzeichnet, daß** ein Bezugswert zur Detektion einer Exspirationsphase (13) vor einem erwarteten Beginn der Exspirationsphase (13) abgesenkt wird.

5. Verfahren nach einem der Ansprüche 1 bis 3, **dadurch gekennzeichnet, daß** ein Bezugswert zur Detektion einer Inspirationsphase (14) vor einem erwarteten Beginn der Inspirationsphase (14) abgesenkt wird.

6. Verfahren nach einem der Ansprüche 1 bis 5, **dadurch gekennzeichnet, daß** der Bezugswert in Abhängigkeit von einem linearen Wahrscheinlichkeitsverlauf verändert wird.

7. Verfahren nach einem der Ansprüche 1 bis 5, **dadurch gekennzeichnet, daß** der Bezugswert in Abhängigkeit von einem nicht linearen Wahrscheinlichkeitsverlauf verändert wird.

8. Verfahren nach einem der Ansprüche 1 bis 7, **dadurch gekennzeichnet, daß** der Bezugswert in Abhängigkeit von einer Mittelwertbildung verändert wird.

9. Verfahren nach einem der Ansprüche 1 bis 8, **dadurch gekennzeichnet, daß** zur Veränderung des Bezugswertes eine erfaßte Dauer von mindestens zwei Inspirationsphasen (14) ausgewertet wird.

10. Verfahren nach einem der Ansprüche 1 bis 8, **dadurch gekennzeichnet, daß** zur Veränderung des Bezugswertes eine erfaßte Dauer von mindestens zwei Exspirationsphasen (13) ausgewertet wird.

11. Verfahren nach einem der Ansprüche 1 bis 10, **dadurch gekennzeichnet, daß** zur Veränderung des Bezugswertes eine Streuung mindestens eines Meßwertes ausgewertet wird.

12. Verfahren nach einem der Ansprüche 1 bis 11, **dadurch gekennzeichnet, daß** eine adaptive Veränderung des Bezugswertes ausschließlich innerhalb vorgegebener Variationsgrenzen durchgeführt wird.

13. Verfahren nach einem der Ansprüche 1 bis 12, **dadurch gekennzeichnet, daß** der Bezugswert höchstens bis zu einem minimalen Triggerwert abgesenkt wird.

14. Verfahren nach einem der Ansprüche 1 bis 13, **dadurch gekennzeichnet, daß** der Bezugswert höchstens bis zu einem maximalen Triggerwert erhöht wird.

15. Verfahren nach einem der Ansprüche 1 bis 14, **dadurch gekennzeichnet, daß** zur Änderung des Bezugswertes ein neuronales Netz (27) verwendet wird.

16. Verfahren nach einem der Ansprüche 1 bis 14, **dadurch gekennzeichnet, daß** zur Änderung des Bezugswertes eine Fuzzy-Logik (26) verwendet wird.

17. Verfahren nach einem der Ansprüche 1 bis 16, **dadurch gekennzeichnet, daß** die adaptive Veränderung des Bezugswertes im Zusammenhang mit dem Betrieb eines Beatmungsgerätes für CPAP-Anwendungen durchgeführt wird.

**18.** Verfahren nach einem der Ansprüche 1 bis 16, **dadurch gekennzeichnet, daß** die adaptive Veränderung des Bezugswertes im Zusammenhang mit dem Betrieb eines Beatmungsgerätes für COPD-Anwendungen durchgeführt wird.

**19.** Verfahren nach einem der Ansprüche 1 bis 16, **dadurch gekennzeichnet, daß** die adaptive Veränderung des Bezugswertes im Zusammenhang mit dem Betrieb eines Beatmungsgerätes für Sauerstofftherapien durchgeführt wird.

**20.** Verfahren nach einem der Ansprüche 1 bis 19, **dadurch gekennzeichnet, daß** bei der adaptiven Veränderung des Bezugswertes Druckmeßwerte ausgewertet werden.

**21.** Verfahren nach einem der Ansprüche 1 bis 19, **dadurch gekennzeichnet, daß** bei der adaptiven Veränderung des Bezugswertes Strömungsmengenmeßwerte ausgewertet werden.

**22.** Verfahren nach einem der Ansprüche 1 bis 21, **dadurch gekennzeichnet, daß** als Triggersignal unmittelbar ein meßtechnisch erfaßter Signalverlauf verwendet wird.

**23.** Verfahren nach einem der Ansprüche 1 bis 21, **dadurch gekennzeichnet, daß** als Triggersignal ein aus einem meßtechnisch erfaßten Signal abgeleitetes Signal verwendet wird.

**24.** Verfahren nach Anspruch 23, **dadurch gekennzeichnet, daß** als Triggersignal die dritte Potenz der ersten Ableitung eines Meßsignals zum Volumenfluß an Atemgas verwendet wird.

**25.** Vorrichtung zur Beatmung, die eine Abgabesteuerung für Atemgas aufweist und bei der die Abgabesteuerung an einen Sensor für ein Meßsignal angeschlossen und mit einer Triggersignalauswertung versehen ist, die ein zum Meßsignal korrespondierendes Triggersignal mit einem Bezugswert vergleicht, sowie bei der die Abgabesteuerung mit einem Stellelement zur Beeinflussung einer Atemgasströmung verbunden ist, **dadurch gekennzeichnet, daß** die Triggersignalauswertung (22) einen Bezugswertadapter (24) zur zeitlichen Veränderung des Bezugswertes in Abhängigkeit von einem zeitlichen Verlauf aufweist.

**26.** Vorrichtung nach Anspruch 25, **dadurch gekennzeichnet, daß** der Sensor (20) als ein Drucksensor ausgebildet ist.

**27.** Vorrichtung nach Anspruch 25, **dadurch gekennzeichnet, daß** der Sensor (20) als ein Strömungsmengensensor ausgebildet ist.

**28.** Vorrichtung nach einem der Ansprüche 25 bis 27, **dadurch gekennzeichnet, daß** die Abgabesteuerung (19) einen Mittelwertbilder zur Signalaufbereitung aufweist.

**29.** Vorrichtung nach einem der Ansprüche 25 bis 28, **dadurch gekennzeichnet, daß** die Abgabesteuerung (19) zur Veränderung des Bezugswertes ein neuronales Netz (27) aufweist.

**30.** Vorrichtung nach einem der Ansprüche 25 bis 28, **dadurch gekennzeichnet, daß** die Abgabesteuerung (19) zur Veränderung des Bezugswertes eine Fuzzy-Logik (26) aufweist.

**31.** Vorrichtung nach einem der Ansprüche 25 bis 30, **dadurch gekennzeichnet, daß** die Abgabesteuerung (19) als Teil eines Gerätes zur Durchführung einer CPAP-Beatmung ausgebildet ist.

**32.** Vorrichtung nach einem der Ansprüche 25 bis 30, **dadurch gekennzeichnet, daß** die Abgabesteuerung (19) als Teil eines Gerätes zur Durchführung einer COPD-Beatmung ausgebildet ist.

**33.** Vorrichtung nach einem der Ansprüche 25 bis 30, **dadurch gekennzeichnet, daß** die Abgabesteuerung (19) als Teil eines Gerätes zur Durchführung einer Sauerstofftherapie ausgebildet ist.

$\underline{\text{FIG.1}}$

FIG.2

FIG.3

EP 1 346 743 A1

FIG.4

FIG.5

Start

Triggerschwelle = Maximum

Einlesen Signale

Kriterium INSP.?

Kriterium EXSP ?

Time = 0

Time = 0

Einlesen "Erwartete Insp.-Zeit"

Einlesen "Erwartete Exsp.-Zeit"

Einlesen Signale

Einlesen Signale

Berechne Schwellwert

Berechne Schwellwert

Kriterium EXSP.?

Speichern Inspirationszeit

Kriterium INSP.?

Speichern Exspirationszeit

Time ++

Time ++

J

N

N

J

J

N

J

N

**Europäisches Patentamt**

# EUROPÄISCHER TEILRECHERCHENBERICHT

der nach Regel 45 des Europäischen Patentübereinkommens für das weitere Verfahren als
europäischer Recherchenbericht gilt

Nummer der Anmeldung

EP 03 00 2456

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X | WO 92 11054 A (PURITAN BENNETT CORP) 9. Juli 1992 (1992-07-09) * Seite 8, Zeile 14 - Seite 9, Zeile 27; Abbildungen 1-6 * --- | 25,26, 28,31-33 | A61M16/00 |
| X | US 6 305 372 B1 (SERVIDIO JOHN L) 23. Oktober 2001 (2001-10-23) * Spalte 4, Zeile 46 - Spalte 5, Zeile 20 * * Spalte 8, Zeile 14 - Zeile 29 * * Spalte 9, Zeile 16 - Zeile 30; Abbildungen * --- | 25-28, 31-33 | |
| X | US 2001/027792 A1 (BERTHON-JONES MICHAEL ET AL) 11. Oktober 2001 (2001-10-11) * Absatz [0045] - Absatz [0056]; | 25-28, 30-33 | |
| Y | Abbildungen * --- -/-- | 29 | |

| | | RECHERCHIERTE SACHGEBIETE (Int.Cl.7) |
|---|---|---|
| | | A61M |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

1-24

Grund für die Beschränkung der Recherche:

Artikel 52 (4) EPÜ - Verfahren zur therapeutischen Behandlung des menschlichen oder tierischen Körpers

| Recherchenort | Abschlußdatum der Recherche | Prüfer |
|---|---|---|
| DEN HAAG | 18. Juni 2003 | Zeinstra, H |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

**Europäisches**
**Patentamt**

**EUROPÄISCHER**
**TEILRECHERCHENBERICHT**

Nummer der Anmeldung

EP 03 00 2456

| | EINSCHLÄGIGE DOKUMENTE | | | KLASSIFIKATION DER ANMELDUNG (Int.Cl.7) |
|---|---|---|---|---|
| Kategorie | Kennzeichnung des Dokuments mit Angabe, soweit erforderlich der maßgeblichen Teile | Betrifft Anspruch | | |
| X | WO 96 11717 A (BIRD PRODUCTS CORP) 25. April 1996 (1996-04-25) * Seite 14, Zeile 27 - Seite 16, Zeile 8; Abbildungen 1,2 * * Seite 19, Zeile 25 - Seite 20, Zeile 30 * * Seite 24, Zeile 4 - Zeile 21 * --- | 25-28, 31-33 | | |
| Y | US 2001/039950 A1 (MAURER JORG ET AL) 15. November 2001 (2001-11-15) | 29 | | |
| A | * Ansprüche 27,28 * ----- | 30 | | |

RECHERCHIERTE
SACHGEBIETE (Int.Cl.7)

EPO FORM 1503 03.82 (P04C12)

## ANHANG ZUM EUROPÄISCHEN RECHERCHENBERICHT
## ÜBER DIE EUROPÄISCHE PATENTANMELDUNG NR.

EP 03 00 2456

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-06-2003

| Im Recherchenbericht angeführtes Patentdokument | | Datum der Veröffentlichung | Mitglied(er) der Patentfamilie | | Datum der Veröffentlichung |
|---|---|---|---|---|---|
| WO 9211054 | A | 09-07-1992 | US | 5134995 A | 04-08-1992 |
| | | | AT | 190230 T | 15-03-2000 |
| | | | AU | 686034 B2 | 29-01-1998 |
| | | | AU | 4071195 A | 18-04-1996 |
| | | | AU | 6371798 A | 30-07-1998 |
| | | | AU | 663054 B2 | 28-09-1995 |
| | | | AU | 8215491 A | 22-07-1992 |
| | | | CA | 2097502 A1 | 22-06-1992 |
| | | | CA | 2206784 A1 | 22-06-1992 |
| | | | DE | 69132030 D1 | 13-04-2000 |
| | | | DE | 69132030 T2 | 19-10-2000 |
| | | | DK | 563044 T3 | 14-08-2000 |
| | | | EP | 0563044 A1 | 06-10-1993 |
| | | | EP | 0968734 A2 | 05-01-2000 |
| | | | ES | 2145739 T3 | 16-07-2000 |
| | | | JP | 2927958 B2 | 28-07-1999 |
| | | | JP | 6503484 T | 21-04-1994 |
| | | | WO | 9211054 A1 | 09-07-1992 |
| | | | US | 5549106 A | 27-08-1996 |
| | | | US | 5794614 A | 18-08-1998 |
| | | | US | 5845636 A | 08-12-1998 |
| | | | US | 5259373 A | 09-11-1993 |
| US 6305372 | B1 | 23-10-2001 | US | 5927274 A | 27-07-1999 |
| | | | US | 5598838 A | 04-02-1997 |
| US 2001027792 | A1 | 11-10-2001 | AU | 757163 B2 | 06-02-2003 |
| | | | AU | 2489601 A | 13-09-2001 |
| | | | EP | 1132106 A2 | 12-09-2001 |
| | | | JP | 2001286564 A | 16-10-2001 |
| WO 9611717 | A | 25-04-1996 | CA | 2201698 A1 | 03-10-1998 |
| | | | US | 2002005197 A1 | 17-01-2002 |
| | | | BR | 9509306 A | 23-12-1997 |
| | | | EP | 0800412 A1 | 15-10-1997 |
| | | | JP | 10507116 T | 14-07-1998 |
| | | | WO | 9611717 A1 | 25-04-1996 |
| | | | US | 5694926 A | 09-12-1997 |
| | | | US | 5881722 A | 16-03-1999 |
| | | | US | 5868133 A | 09-02-1999 |
| | | | AT | 235280 T | 15-04-2003 |
| | | | DE | 69530117 D1 | 30-04-2003 |
| | | | EP | 1205206 A2 | 15-05-2002 |
| | | | EP | 1205203 A2 | 15-05-2002 |
| US 2001039950 | A1 | 15-11-2001 | DE | 10014427 A1 | 04-10-2001 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**

EP 03 00 2456

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-06-2003

| Im Recherchenbericht angeführtes Patentdokument | Datum der Veröffentlichung | Mitglied(er) der Patentfamilie | Datum der Veröffentlichung |
|---|---|---|---|
| US 2001039950 A1 | | EP 1136094 A2 | 26-09-2001 |

EPO FORM P0461

Für nähere Einzelheiten zu diesem Anhang : siehe Amtsblatt des Europäischen Patentamts, Nr.12/82